# EUROPÄISCHE PATENTSCHRIFT

(11) **EP 1 827 228 B1**
(45) Veröffentlichungstag und Bekanntmachung des Hinweises auf die Patenterteilung: **01.01.2014**
(21) Anmeldenummer: 05802034.8
(22) Anmeldetag: 07.11.2005
(51) Int. Cl.: A61B 5/103

(54) **VORRICHTUNG UND VERFAHREN ZUM BESTIMMEN WENIGSTENS EINES CHARAKTERISTISCHEN PUNKTES EINES ORTHOPÄDISCH ZU VERMESSENDEN GELENKS**
DEVICE AND METHOD FOR DETERMINING AT LEAST ONE CHARACTERISTIC POINT OF JOINT TO BE ORTHOPEDICALLY MEASURED
DISPOSITIF ET PROCÉDÉ PERMETTANT DE DÉTERMINER AU MOINS UN POINT CARACTÉRISTIQUE D'UNE ARTICULATION À MESURER PAR VOIE ORTHOPÉDIQUE

(30) Priorität: 16.11.2004 DE 102004055234
(43) Veröffentlichungstag der Anmeldung: 05.09.2007
(73) Patentinhaber: Fraunhofer-Gesellschaft zur Förderung der angewandten Forschung e.V., 80686 München (DE)
(72) Erfinder: LISSEK, Karsten, 80687 München (DE); SCHNEIDER, Urs, 70174 Stuttgart (DE); VON LÜBTOW, Kai, 70597 Stuttgart (DE); HAID, Markus, 70567 Stuttgart (DE); FUCHS, Alfons, 69221 Dossenheim (DE); BAUERNFEIND, Roland, 81669 München (DE)
(74) Vertreter: Thum, Bernhard
(86) Internationale Anmeldenummer: PCT/EP2005/011917
(87) Internationale Veröffentlichungsnummer: WO 2006/053650

(56) Entgegenhaltungen:
- WO-A-03/047430
- DE-A1- 10 014 397
- US-A- 4 436 099
- US-B1- 6 692 447

## Beschreibung

Die vorliegende Erfindung betrifft eine Vorrichtung zum Bestimmen wenigstens eines charakteristischen Punktes eines orthopädisch zu vermessenden, zwei Glieder miteinander verbindenden Gelenks, wobei die Vorrichtung ein erstes Kopplungselement, das an einem an das Gelenk angrenzenden ersten Glied anbringbar ist, ein relativ zu dem ersten Kopplungselement bewegbares zweites Kopplungselement, das an einem an das Gelenk angrenzenden zweiten Glied anbringbar ist, und eine Messeinrichtung zum Bestimmen der Lage des ersten Kopplungselements relativ zu dem zweiten Kopplungselement umfasst.

In der Orthopädietechnik gibt es verschiedene Anwendungsfälle, in denen ein Gelenk der menschlichen Extremitäten zu vermessen ist. Insbesondere ist es im Bereich der Gelenkorthetik oder Gelenkprothetik erforderlich, das dynamische Verhalten von Gelenken der menschlichen Extremitäten zu ermitteln. So ist es beispielsweise erforderlich, Orthesen, die eine externe Stabilisierungshilfe zur mechanischen und funktionellen Stabilisierung von Gelenken darstellen, speziell auf das jeweilige zu unterstützende Gelenk des Patienten anzupassen. Ein bevorzugter Applikationsfall von Orthesen sind Kniegelenkorthesen. Diese werden zur Stabilisierung des Kniegelenks bei krankhafter oder operativ bedingter Schwächung ebenso eingesetzt, wie bei Leistungssportlern zur Vermeidung von Überbelastungen. Gerade das Kniegelenk verhält sich allerdings nicht wie ein einfaches Scharniergelenk mit einer singulären Drehachse, sondern eher wie ein polyzentrisches Gelenk mit einer Mehrzahl von Drehpunkten, deren Drehpunktlage sich bei einer Relativbewegung von Oberschenkel und Unterschenkel zueinander ständig verändern. Obgleich es zwischenzeitlich verschiedene Ansätze gibt, im Bereich der Orthetik sowie der Prothetik polyzentrische Gelenke einzusetzen, die das tatsächliche Verhalten des jeweiligen zu versorgenden menschlichen Gelenks mehr oder weniger gut nachbilden, werden weiterhin im Wesentlichen monozentrische Gelenke in Orthesen bzw. Prothesen eingesetzt.

Sowohl monozentrische als auch polyzentrische Orthesen bzw. Prothesen müssen an den jeweiligen Patienten individuell angepasst werden. Eine Fehlanpassung einer Orthese kann beispielsweise zur Folge haben, dass die Orthese nicht die gewünschte mechanische und funktionelle Stabilisierung des jeweiligen Gelenks gewährleistet. Ferner kann eine Fehlanpassung einer Orthese sogar zu Folgeschäden für das Gelenk führen, weil dieses Gelenk durch den von der Orthese an sich zur Stabilisierung des Gelenks bereitgestellten Widerstand gegen bestimmte Bewegungen zusätzlich belastet wird. Zur Anpassung der Orthese bzw. Prothese werden daher bislang ein Kompromissdrehpunkt oder/und eine den Verlauf von Momentandrehpunkten zu verschiedenen Beugewinkeln des Gelenks wiederspiegelnde Polkurve ermittelt. Nach Maßgabe des ermittelten Kompromissdrehpunktes bzw. der Polkurve wird dann die Orthese bzw. die Prothese von einem Orthopädietechniker eingestellt. Zur Ermittlung des Kompromissdrehpunktes wird insbesondere das Verfahren nach Nietert eingesetzt, das dem Fachmann allgemein bekannt ist. Daraus ergibt sich ein Bereich, in dem der Kompromissdrehpunkt zu erwarten ist. Es hat sich allerdings gezeigt, dass eine derartige Bestimmung nicht hinreichend genau ist, um beispielsweise eine Orthese zuverlässig unter Vermeidung von Fehlanpassungen an jeden Patienten anzupassen.

In der Vergangenheit wurde die Vermessung von Rotationsachsen in Gelenken hauptsächlich in aufwendigen klinischen Studien durchgeführt. Vorzugsweise wurden dabei in Knochen von Leichenpräparaten Marker gesetzt und deren Bewegung photografisch sowie röntgenologisch verfolgt. Diese Maßnahmen führen zu allgemeinen Erkenntnissen hinsichtlich der Lage von Kompromissdrehpunkt und Polkurven an verschiedenen Gelenken, helfen aber nicht zur individuellen Anpassung einer Orthese bzw. Prothese an den jeweiligen Patienten weiter.

Zur speziellen Anpassung von Orthesen an Patienten werden seit langer Zeit Goniometer eingesetzt. Damit lässt sich beispielsweise das Bewegungsausmaß eines Gelenks bestimmen. Allerdings kann durch den Einsatz eines Goniometers keinerlei Aussage über die Lage der Rotationsachsen gemacht werden. Zum Aufbau und zur Funktionsweise eines orthopädisch eingesetzten Goniometers wird auf das Dokument WO94/07108 verwiesen. Auch der Stand der Technik gemäß US 4,306,571 beschreibt die Möglichkeit des Einsatzes eines Mehrfach-Goniometers zur Gewinnung von Daten bei der Belastung eines Kniegelenks.

Aus der europäischen Patentanmeldung EP 1 454 584 A1 ist ein anderer Ansatz zur Vermessung von Patienten bekannt. Dabei wird ein Patient auf einer auf Messzellen gelagerten Tragplatte positioniert und mittels Laserstrahlen vermessen. Dieser Stand der Technik sieht insbesondere vor, nach Maßgabe der Erfassung über die Tragplatte eine Kraftwirkungslinie für einen Patienten zu erzeugen, anhand der eine Prothese oder eine Orthese angepasst werden kann.

Das Dokument DE 10014397 A1 beschreibt ein Gerät zur Vermessung von Gelenkwinkeln. Das Gerät besteht aus zwei Schienen, die durch ein vorzugsweise fixierbares Drehgelenk mit Winkelmesser verbunden sind. An diese Schienen sind Anlageelemente angebracht, von denen sich mindestens eine axial zur Schiene verschieben lässt. Zur Anwendung des Gerätes werden die zu vermessenden Körperglieder, die durch ein gemeinsames Gelenk verbunden sind, in die zu vermessende Stellung gebracht. Die Anlageelemente werden nun an definierte anatomische Punkte an den Körpergliedern so angelegt, dass der gegenwärtige Winkel zwischen den Schienen dem Gelenkwinkel zwischen den Körpergliedern entspricht. Eine Fixierung des Drehgelenks des Gerätes erleichtert die Wegnahme des Geräts vom Körper und das Ablesen des Gelenkwinkels, ohne den Winkel zwischen den Schienen zu verändern.

Ferner beschreibt das Dokument US 4,436,009 ein ähnliches Gerät zur Vermessung der relativen Winkelverschiebung zwischen zwei Körpergliedern, die durch ein gemeinsames Gelenk verbunden sind. Bei diesem Gerät sind zwei starre Stangen mit einer Messdose verbunden, wobei eine der Stangen durch ein an der Dose befestigtes Gelenk drehbar ist und die andere Stange an der Dose fixiert ist. Die Stangen werden jeweils an einem der Körperglieder durch Halterungen befestigt, in denen die Stangen rotieren können und parallel zur Längsachse des betreffenden Körpergliedes verschiebbar sind. In der Messdose befindet sich eine Schaltung, die ein elektrisches Signal produziert, das ein Maß für die Winkelverlagerung zwischen den starren Stangen, also auch für die Winkelverlagerung zwischen den Körpergliedern ist. Das elektrische Signal wird über ein Kabel an einen Zähler und ein Display weitergeführt. Das Display wird zweckmäßig räumlich getrennt vom zu vermessenden Körper aufgestellt, so dass die gemessene Winkelverschiebung zwischen den Gliedern einfach abgelesen werden kann.

Es ist Aufgabe der vorliegenden Erfindung, eine Vorrichtung und ein Verfahren der eingangs bezeichneten Art bereitzustellen, mit welchen es bei einfacher Handhabung möglich ist, einen charakteristischen Punkt eines orthopädisch zu vermessenden Gelenks eines Patienten mit hinreichender Genauigkeit zu bestimmen.

Diese Aufgabe wird durch eine Vorrichtung der eingangs bezeichneten Art gelöst, bei der die Messeinrichtung wenigstens einen Bezugspunkt aufweist, der fest mit einem von erstem und zweitem Kopplungselement verbunden ist, und ferner eine Bezugspunkt-Erfasssungseinrichtung aufweist, die fest mit dem jeweils anderen von erstem und zweitem Kopplungselement verbunden ist, wobei die Bezugspunkt-Erfassungseinrichtung bei einer Bewegung von erstem und zweitem Glied zueinander eine Mehrzahl von Positionen des Bezugspunkts relativ zu der Bezugspunkt-Erfassungseinrichtung erfasst und daraus den wenigstens einen charakteristischen Punkt als wenigstens einen virtuellen Drehpunkt für das zu vermessende Gelenk ermittelt.

Die erfindungsgemäße Vorrichtung lässt sich direkt an den durch das zu vermessende Gelenk miteinander verbundenen Gliedern anbringen, d.h. an diese anhalten oder daran befestigen. Nach der Anbringung der Kopplungselemente der erfindungsgemäβen Vorrichtung an den Gliedern lassen sich diese unter Beugung des Gelenks relativ zueinander bewegen, wobei das Gelenk seine natürlichen Bewegungen ausführt. So ist es möglich, die Relativbewegung der beiden Glieder zueinander in im Wesentlichen unbelasteter Lage des Gelenks sowie unter voller Belastung des Gelenks mittels der erfindungsgemäßen Vorrichtung zu erfassen. Dies geschieht dadurch, dass sich der wenigstens eine Bezugspunkt, der fest mit dem einen von erstem und zweitem Kopplungselement verbunden ist, entsprechend der Gelenkbewegung mit dem jeweils zugeordneten Kopplungselement mitbewegt. Diese Bewegung wird durch die Bezugspunkt-Erfassungseinrichtung zumindest an mehreren Stellen punktuell erfasst. Aus diesen Momentanpositionen des wenigstens einen Bezugspunkts lässt sich dann als der charakteristische Punktder virtuelle Drehpunkt (in Fachkreisen oftmals auch als Kompromissdrehpunkt, Kompromissdrehzentrum oder Kompromissdrehachse bezeichnet) bestimmen. Die somit gewonnenen Erkenntnisse über die Lage des charakteristischen Punktes, d.h. des Kompromissdrehzentrums, lassen sich dann von einem Orthopäden oder einem Orthopädietechniker zur Anpassung einer Orthese oder einer Prothese nutzen. So kann beispielsweise zur Anpassung einer monozentrischen Orthese deren Drehgelenkachse in Übereinstimmung mit der durch die erfindungsgemäße Vorrichtung ermittelten Kompromissdrehachse gebracht werden, um in bestmöglicher Art und Weise die mechanische Stabilisierungsfunktion der Orthese für das zu stabilisierende Gelenk auszunützen. Ferner kann mittels der erfindungsgemäβen Vorrichtung ein charakteristischer Punkt, d.h. ein Kompromissdrehzentrum, für ein zu vermessendes gesundes Gelenk bestimmt werden und nach Maßgabe der erhaltenen Messdaten eine korrespondierende Anpassung einer Gelenkprothese für eine prothetisch zu versorgende Extremität vorgenommen werden.

Die Erfindung schafft also die Möglichkeit, mit verhältnismäßig einfachen technischen Mitteln und unter geringem messtechnischem Aufwand ein Gelenk hinreichend genau orthopädisch zu vermessen, wobei die gewonnenen Messdaten zur orthetischen oder prothetischen Versorgung eines Patienten weiter genutzt werden können.

Eine Alternative der Erfindung zum Bestimmen einer Mehrzahl charakteristischer Punkte sieht vor, dass die Bezugspunkt-Erfassungseinrichtung aus der Mehrzahl von Positionen des Bezugspunkts eine Mehrzahl charakteristischer Punkte als eine Polkurve für das zu vermessende Gelenk ermittelt. Die Polkurve (auch Zentrode genannt) spiegelt den Verlauf der Momentandrehpunkte bei verschiedenen Beugewinkein des jeweiligen Gelenks wieder. Nach Maßgabe der ermittelten Polkurve lässt sich dann beispielsweise eine polyzentrische Orthese oder Prothese einstellen, so dass die Polkurve der Orthese weitgehend mit der Polkurve des orthetisch zu versorgenden Gelenks übereinstimmt oder dass die Polkurve der polyzentrischen Prothese für eine prothetisch zu versorgende Extremität eines Patienten an die Polkurve des gesunden Gelenks der jeweils anderen Extremität des Patienten entsprechend zur Vermeidung von asymmetrischen Belastungen des Patienten angepasst ist.

Eine Weiterbildung der Erfindung sieht vor, dass die Bezugspunkt-Erfassungseinrichtung aus der Mehrzahl von Positionen des Bezugspunkts eine Bewegungsbahn des jeweiligen Bezugspunkts erfasst. Es ist festzuhalten, dass mit zunehmender Anzahl der erfassten Positionen des Bezugspunktes die über das orthopädisch zu vermessende Gelenk gewonnenen Erkenntnisse detaillierter werden und damit eine genauere Bestimmung des bzw. der charakteristischen Punkte dieses Gelenks möglich ist. Bei der erfassten Bewegungsbahn kann es sich um eine zweidimensionale (in eine Ebene projizierte) Kurve oder um eine dreidimensionale Raumkurve handeln.

Bei einer bevorzugten Ausführungsform der Erfindung ist eine Verarbeitungseinrichtung vorgesehen, die mit der Bezugspunkt-Erfassungseinrichtung zur Übermittlung von Daten gekoppelt oder koppelbar ist. Die Verarbeitungseinrichtung verarbeitet die von der Bezugspunkt-Erfassungseinrichtung erhaltenen Daten in vorgegebener Weise. Die Verarbeitungseinrichtung kann separat von der an dem Gelenk anlegbaren oder befestigbaren Vorrichtung oder auch integral mit dieser ausgebildet sein. Vorzugsweise ist bei einer Weiterbildung der Erfindung vorgesehen, dass die Verarbeitungseinrichtung aus den von der Bezugspunkt-Erfassungseinrichtung erhaltenen Daten, insbesondere aus der Bewegungsbahn des jeweiligen Bezugspunkts, den wenigstens einen charakteristischen Punkt oder die Mehrzahl charakteristischer Punkte ermittelt.

Insbesondere zur Einstellung von polyzentrischen Gelenken sind detaillierte Untersuchungen erforderlich, die über die Ermittlung eines singulären charakteristischen Punkts, beispielsweise des Kompromissdrehzentrums, hinausgehen und es erfordern, dass die Verarbeitungseinrichtung aus den von der Bezugspunkt-Erfassungseinrichtung erhaltenen Daten, insbesondere aus der Bewegungsbahn des jeweiligen Bezugspunkts, die Mehrzahl charakteristischer Punkte für das zu vermessende Gelenk ermittelt.

Wie vorstehend bereits dargelegt, werden die über die erfindungsgemäße Vorrichtung gewonnenen Erkenntnisse über den charakteristischen Punkt des orthopädisch zu vermessenden Gelenks in einem bevorzugten Anwendungsfall zur Einstellung einer Orthese für das Gelenk genutzt. Am einfachsten geschieht dies dadurch, dass die Orthese direkt in unmittelbarer Nähe zu dem Gelenk angepasst wird. Hierzu ist es erforderlich, dass der ermittelte charakteristische Punkt, beispielsweise der virtuelle Drehpunkt (Kompromissdrehpunkt) an dem orthopädisch zu vermessenden Gelenk sichtbar gemacht wird. Gemäß einer Weiterbildung der Erfindung ist hierfür eine optische Ausgabeeinrichtung vorgesehen, mittels der der wenigstens eine ermittelte charakteristische Punkt des Gelenks optisch an dem zu vermessenden Gelenk anzeigbar ist. Dies kann beispielsweise dadurch geschehen, dass die Ausgabeeinrichtung eine gewinkelte Anordnung einer Mehrzahl von Lichtquellen umfasst, mittels denen vorzugsweise gerichtete Lichtstrahlen aussendbar sind, wobei der wenigstens eine ermittelte virtuelle Drehpunkt durch einem Kreuzungspunkt zweier beiden Lichtstrahlen anzeigbar ist. Alternativ kann der ermittelte charakteristische Punkt des Gelenks auch durch einen oder mehrere Laserstrahlen angezeigt werden.

Alternativ zur Anzeige des wenigstens einen ermittelten virtuellen Drehpunkts durch sich in einem Kreuzungspunkt kreuzende Lichtstrahlen ist es auch möglich, dass die Ausgabeeinrichtung eine Anordnung einer Mehrzahl von Lichtquellen aufweist, die Anlagepunkte insbesondere zum Anlegen einer Lehrenanordnung, insbesondere einer Linealanordnung anzeigen, wobei durch entsprechendes Anlegen der Lehrenanordnung der wenigstens eine ermittelte virtuelle Drehpunkt an dem Gelenk anzeigbar ist. So können einzelne Lichtpunkte Koordinaten für den virtuellen Drehpunkt anzeigen, so dass durch Anlage eines oder mehrerer Lineale durch einen Orthopädietechniker oder dergleichen anhand der angezeigten Koordinaten mittels der Lineale der ermittelte virtuelle Drehpunkt an dem Gelenk anzeigbar und markierbar ist. Alternativ kann der Orthopädietechniker ohne Lineale per Augenmaß anhand der Anzeigepunkte den virtuellen Drehpunkt an dem Gelenk antragen.

Alternativ zu einer optischen Anzeigeeinrichtung mittels Lichtquellen sieht eine Weiterbildung der Erfindung eine elektromechanische Ausgabeeinrichtung vor, die eine elektromechanisch verlagerbare Markierung aufweist, wobei der wenigstens eine ermittelte virtuelle Drehpunkt durch entsprechende Positionierung der elektromechanisch verlagerbaren Markierung an dem Gelenk anzeigbar ist. Beispielsweise kann die Markierung über ein oder mehrere Linearstellglieder innerhalb eines bestimmten Feldes elektromechanisch verlagert werden und nach Maßgabe des ermittelten charakteristischen Punktes dann die Markierung durch elektromechanische Ansteuerung der Stellglieder an dem wenigstens einen ermittelten virtuellen Drehpunkt zur Markierung verharren.

Bei einer vorteilhaften Weiterbildung der Erfindung ist eine Markiervorrichtung, insbesondere ein Stift vorgesehen, mittels der an dem wenigstens einen ermittelten virtuellen Drehpunkt nach entsprechender elektromechanischer Verlagerung der Markierung an dem Gelenk eine Farbmarkierung anbringbar ist. Somit kann die erfindungsgemäße Vorrichtung selbsttätig eine Markierung an dem orthetisch oder prothetisch zu versorgenden Gelenk des Patienten anbringen, die dann für spätere Anpassungszwecke einer Orthese bzw. Prothese genutzt werden kann.

Vorstehend wurde allgemein davon gesprochen, dass mittels der Bezugspunkt-Erfassungseinrichtung eine Erfassung der aktuellen Position des Bezugspunkts möglich ist. Dies kann beispielsweise dadurch geschehen, dass die Bezugspunkt-Erfassungseinrichtung die Position des wenigstens einen Bezugspunkts optisch erfasst. Alternativ oder zusätzlich hierzu kann vorgesehen sein, dass die Bezugspunkt-Erfassungseinrichtung elektronisch die Position des wenigstens einen Bezugspunkts erfasst. Beispielsweise lässt sich die Position des Bezugspunkts magnetisch über Hall-Sensoren ermitteln. Alternativ ist es auch denkbar, die beiden Kopplungselemente über eine Mehrgelenkanordnung miteinander zu verbinden, wobei über Dreh- oder Linearpositionsgeber die exakte Relativposition des ersten Kopplungselements relativ zu dem zweiten Kopplungselement bestimmbar ist. Bei derartigen Linear- bzw. Drehpositionsgebern kann es sich beispielsweise um entsprechende Linear- oder Drehpotentiometer handeln. Grundsätzlich ist festzustellen, dass jede technische Möglichkeit zur Bestimmung der Relativlage des ersten Kopplungselements relativ zu dem zweiten Kopplungselement, insbesondere der Bestimmung der Relativlage wenigstens eines mit einem Kopplungselement fest verbundenen Bezugspunkts relativ zu dem anderen Kopplungselement im Rahmen der Erfindung liegt, solange sie hinreichende Genauigkeit bietet.

Die Messgenauigkeit der erfindungsgemäßen Vorrichtung lässt sich weiter dadurch erhöhen, dass wenigstens zwei voneinander beabstandete Bezugspunkte überwacht werden, die fest mit dem einen von erstem und zweitem Kopplungselement verbunden sind. Dabei erfasst die Bezugspunkt-Erfassungseinrichtung bei einer Bewegung von erstem und zweitem Glied zueinander eine Mehrzahl von Positionen der Bezugspunkte, vorzugsweise die Bewegungsbahnen der wenigstens zwei Bezugspunkte relativ zu der Bezugspunkt-Erfassungseinrichtung.

Die Erfindung betrifft ferner ein Verfahren zum Bestimmen wenigstens eines charakteristischen Punktes eines orthopädisch zu vermessenden, zwei Glieder miteinander verbindenden Gelenks, unter Verwendung einer Vorrichtung, insbesondere der vorstehend beschriebene Art, wobei das Verfahren die Schritte umfasst:
- Anbringen eines ersten Kopplungselements an einem an das Gelenk angrenzenden ersten Glied,
- Anbringen eines relativ zu dem ersten Kopplungselement bewegbaren zweiten Kopplungselements an einem an das Gelenk angrenzenden zweiten Glied und
- Bestimmen der Lage des ersten Kopplungselements relativ zu dem zweiten Kopplungselement mittels einer Messeinrichtung.

Bei diesem Verfahren ist erfindungsgemäß vorgesehen, dass die Messeinrichtung wenigstens einen Bezugspunkt aufweist, der fest mit einem von erstem und zweitem Kopplungselement verbunden ist, und ferner eine Bezugspunkt-Erfassungseinrichtung aufweist, die fest mit dem jeweils anderen von erstem und zweitem Kopplungselement verbunden ist, wobei durch die Bezugspunkt-Erfassungseinrichtung bei einer Bewegung von erstem und zweitem Glied zueinander eine Mehrzahl von Positionen des Bezugspunkts relativ zu der Bezugspunkt-Erfassungseinrichtung erfasst werden und daraus der wenigstens eine charakteristische Punkt als ein virtueller Drehpunkt für das zu vermessende Gelenk ermittelt wird.

Die Erfindung betrifft ferner ein alternatives Verfahren zum Bestimmen einer Mehrzahl charakteristischer Punkte, bei dem im Unterschied zu dem vorstehend beschriebenen Verfahren zwar auch durch die Bezugspunkt-Erfassungseinrichtung bei einer Bewegung von erstem und zweitem Glied zueinander eine Mehrzahl von Positionen des Bezugspunkts relativ zu der Bezugspunkt-Erfassungseinrichtung erfasst werden, jedoch daraus die Mehrzahl charakteristischer Punkte als eine Polkurve für das zu vermessende Gelenk ermittelt wird.

Gemäß einer Weiterbildung des erfindungsgemäßen Verfahrens wird eine Bewegungsbahn des jeweiligen Bezugspunktes aus der Mehrzahl von Positionen des Bezugspunkts durch die Bezugspunkt-Erfassungseinrichtung erfasst.

Ferner kann bei einer Weiterbildung der Erfindung der wenigstens eine charakteristische Punkt bzw. die Mehrzahl charakteristischer Punkte aus den von der Bezugspunkt-Erfassungseinrichtung erhaltenen Daten, insbesondere aus der Bewegungsbahn des jeweiligen Bezugspunkts, ermittelt werden.

Ferner kann bei dem erfindungsgemäßen Verfahren vorgesehen sein, dass wenigstens zwei voneinander beabstandete Bezugspunkte verwendet werden, die fest mit dem einen von erstem und zweitem Kopplungselement verbunden sind, wobei bei einer Bewegung von erstem und zweitem Glied zueinander eine Mehrzahl von Positionen der Bezugspunkte, vorzugsweise die Bewegungsbahnen der wenigstens zwei Bezugspunkte relativ zu der Bezugspunkt-Erfassungseinrichtung erfasst werden.

Die Erfindung wird im Folgenden beispielhaft anhand der beiliegenden Figuren erläutert. Es stellen dar:
- Fig.1: eine Übersichtsdarstellung zu einer Messsituation, bei der die erfindungsgemäße Vorrichtung eingesetzt wird;
- Fig.2: eine teilweise geschnittene Vorderansicht eines ersten Ausführungsbeispiels der erfindungsgemäßen Vorrichtung;
- Fig.3: eine Ansicht entsprechend Fig.2 eines gegenüber dieser abgewandelten zweiten Ausführungsbeispiels der erfindungsgemäßen Vorrichtung;
- Fig.4: eine Ansicht entsprechend Fig.3 eines gegenüber dieser abgewandelten dritten Ausführungsbeispiels der erfindungsgemäßen Vorrichtung;
- Fig.5: eine Ansicht entsprechend Fig.2 bis 4 einer vereinfacht ausgebildeten vierten Ausführungsform der erfindungsgemäßen Vorrichtung;
- Fig.6: ein Flussdiagramm zur Erläuterung eines erfindungsgemäßen Verfahrens, bei dem die erfindungsgemäße Vorrichtung eingesetzt wird;
- Fig.7: ein Flussdiagramm zur Erläuterung einer Abwandlung des erfindungemäßen Verfahrens.

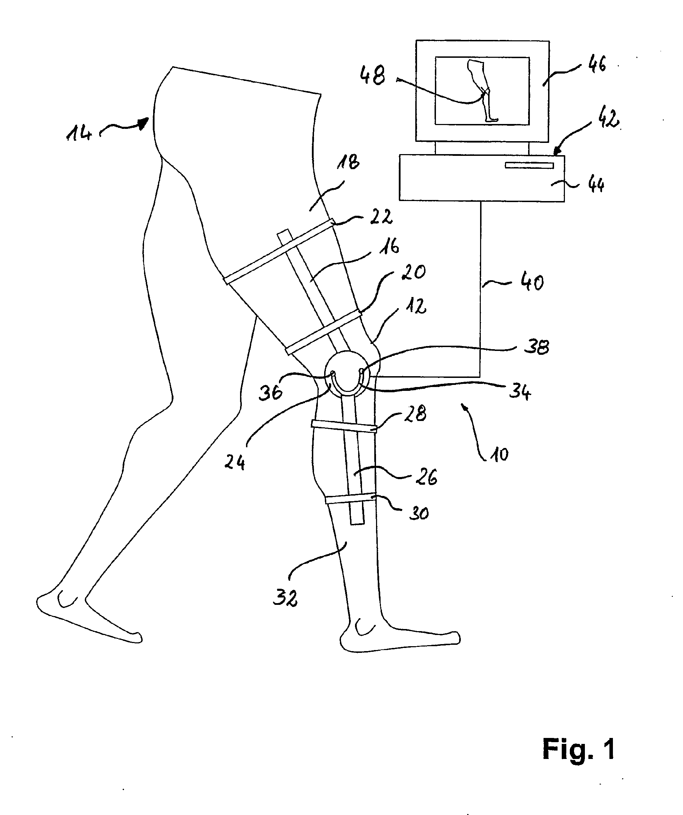

In Fig.1 ist eine typische Messsituation dargestellt, in der eine allgemein mit 10 bezeichnete erfindungsgemäße Vorrichtung eingesetzt wird. Bei dem Einsatzfall gemäß Fig.1 wird das rechte Knie 12 eines Patienten 14 mit der erfindungsgemäßen Vorrichtung 10 hinsichtlich seines dynamischen Verhaltens vermessen. Von dem Patienten 14 sind lediglich die unteren Extremitäten dargestellt.

Die erfindungsgemäße Vorrichtung umfasst ein leistenförmig ausgebildetes erstes Kopplungselement 16, das mit dem Oberschenkel 18 des rechten Beins des Patienten 14 fest verbunden ist. Hierzu ist das erste Kopplungselement 16 über zwei elastische Bänder 20, 22 an dem Oberschenkel 18 des rechten Beins des Patienten 14 an dessen Außenseite festgeklemmt. An dem ersten Kopplungselement 16 ist eine Erfassungs- und Anzeigeeinrichtung 24 fest angebracht. Die erfindungsgemäße Vorrichtung 10 umfasst ein zweites Kopplungselement 26. Das zweite Kopplungselement, das wiederum leistenförmig ausgebildet ist, ist in entsprechender Weise über zwei elastische Bänder 28, 30 mit dem Unterschenkel 32 des rechten Beins des Patienten 14 fest verbunden. An seinem dem ersten Kopplungselement 16 zugewandten Ende weist das zweite Kopplungselement 26 einen fest mit dem Kopplungselement 26 verbundenen Gabelabschnitt 34 auf. An den freien Enden des Gabelabschnitts 34 sind zwei Bezugspunkte 36, 38 vorgesehen. Die Bezugspunkte 36, 38 sind derart ausgebildet, dass sich deren Position mittels der Erfassungs- und Anzeigeeinrichtung 24 vorzugsweise mit einer Genauigkeit von wenigen Millimetern erfassen lässt.

Die Erfassungs- und Anzeigeeinrichtung 24 ist über eine Datenverbindung 40 mit einer Verarbeitungseinrichtung 42 verbunden. Die Verarbeitungseinrichtung 42 umfasst eine Rechnereinheit 44 und eine Anzeigeeinheit 46. Die Verarbeitungseinrichtung 42 empfängt die von der Erfassungseinrichtung 24 ermittelten Positionsdaten der Bezugspunkte 36 und 38, speichert diese und verarbeitet sie weiter.

In der in Fig. 1 gezeigten Messsituation macht der Patient 14 eine Schrittbewegung. Die dabei im rechten Bein ausgeführten Bewegungen, insbesondere die Bewegungen des Kniegelenks 12, die zu einer Relativbewegung von Oberschenkel 18 zum Unterschenkel 32 führen haben eine Relativbewegung der Bezugspunkte 36, 38 relativ zu der Erfassungseinrichtung 24 zur Folge. Diese Relativbewegung der Bezugspunkte 36 und 38, insbesondere deren Bewegungsbahn relativ zu der Erfassungseinrichtung 24 wird von letzterer erfasst und an die Verarbeitungseinrichtung 42 weitergeleitet. Die Verarbeitungseinrichtung 42 analysiert die Bewegungsbahnen der Bezugspunkte 36 und 38 relativ zu der Erfassungseinrichtung 24 und ermittelt daraus wenigstens einen charakteristischen Punkt für das Kniegelenk 12. Bei diesem charakteristischen Punkt handelt es sich beispielsweise um das an der Anzeigeeinrichtung 46 mit einem Kreuz markierte Kompromissdrehzentrum 48, das einen Kompromiss aus der während der Bewegung auftretenden Rotationszentren des Kniegelenks 12 bildet. Nach Maßgabe des ermittelten Kompromissdrehzentrums 48 lässt sich dann beispielsweise eine monozentrische Orthese einstellen, wobei deren Orthesengelenk derart positioniert ist, dass seine Drehachse durch das Kompromissdrehzentrum 48 verläuft.

Nachdem mit Bezug auf Fig.1 das Grundprinzip zum Einsatz der erfindungsgemäßen Vorrichtung erläutert wurde, soll im Folgenden im Einzelnen auf verschiedene Ausführungsbeispiele der erfindungsgemäßen Vorrichtung eingegangen werden. Hierzu werden zur Vermeidung von Wiederholungen und zur Vereinfachung der Beschreibung für gleichwirkende oder gleichartige Komponenten dieselben Bezugszeichen verwendet, wie in Fig.1 jedoch mit Kleinbuchstaben nachgestellt.

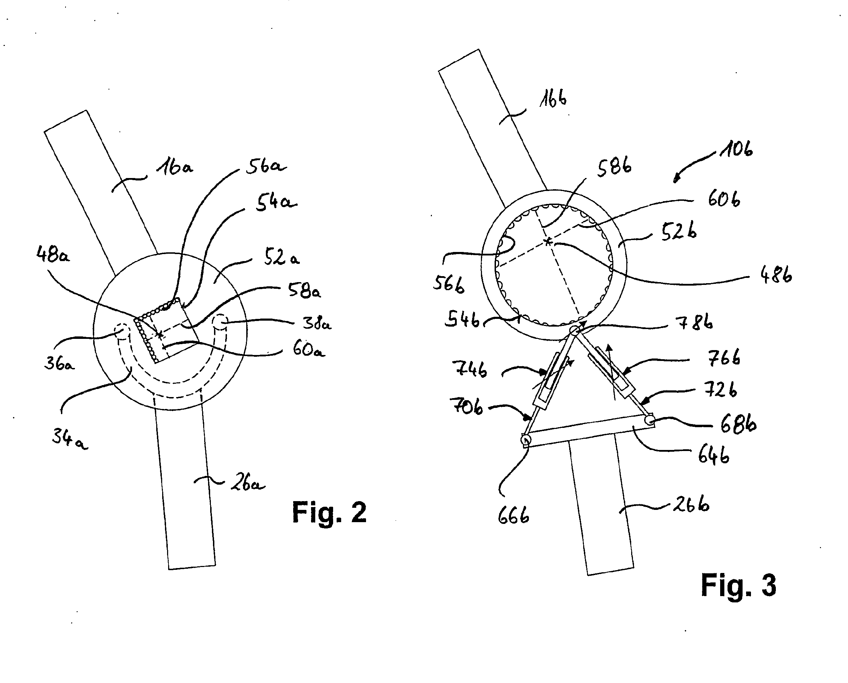

Fig.2 zeigt ein erstes Ausführungsbeispiel der erfindungsgemäßen Vorrichtung, die dort allgemein mit 10a bezeichnet ist. Bei diesem Ausführungsbeispiel sind die beiden Kopplungselemente 16a und 26a verkürzt dargestellt, wobei sie jedoch zum Anlegen oder zur entsprechenden Fixierung an Ober- und Unterschenkel eines Patienten im Wesentlichen dieselbe Länge aufweisen, wie in Fig.1 schematisch dargestellt. Mit dem ersten Kopplungselement 16a ist eine Erfassungs- und Anzeigeeinrichtung 52a fest verbunden. Der Erfassungs- und Anzeigeeinrichtung 52a ist mit einer rechteckigen Durchgangsöffnung 54a versehen. An der Innenumfangsfläche der Durchgangsöffnung 54a ist eine Vielzahl von Leuchtdioden 56a vorgesehen, die im Stile eines karthesischen Koordinatensystems angeordnet sind. Die Leuchtdioden 56a sind derart ausgebildet, dass sie gerichtete Lichtstrahlen aussenden können, wie in Fig.2 durch die strichliert gezeichneten Strahlen 58a und 60a gezeigt. Die Lichtstrahlen 58a und 60a sind derart gerichtet, dass sie in dem in Fig.1 gezeigten Anwendungsfall den in der Draufsicht durch die Durchgangsöffnung 54a erkennbaren Bereich des Kniegelenks 12 bestrahlen können. Somit ist der Kreuzungspunkt der Lichtstrahlen 58a und 60a auf dem Kniegelenk 12 des Patienten 14 zu erkennen.

An dem zweiten Kopplungselement 26a ist, wie in Fig.2 schematisch gezeigt, der Gabelabschnitt 34a angebracht. Der Gabelabschnitt 34a ist innerhalb der Erfassungsund Anzeigeeinrichtung 52a in der Zeichenebene verlagerbar und verschwenkbar aufgenommen. An den freien Enden des Gabelabschnitts 34a sind Emitterelemente 36a, 38a fixiert, so dass diese in fester positionsmäßiger Beziehung zu dem zweiten Kopplungselement 26a stehen. Die Emitterelemente 36a und 38a bilden die Bezugspunkte, deren Position - projiziert in die Zeichenebene - über eine nicht dargestellte Sensorik innerhalb der Erfassungs- und Anzeigeeinrichtung 52a genau erfassbar ist.

Wie vorstehend mit Bezug auf Fig.1 allgemein erläutert, wird bei einer Bewegung des Kniegelenks 12 und einer daraus resultierenden Relativbewegung von den beiden Kopplungselementen 16a und 26a die daraus resultierende Relativbewegung der Bezugspunkte 36a und 38a relativ zu der Erfassungs- und Anzeigeeinrichtung 52a erfasst. Nach Maßgabe der erfassten Bewegungsbahnen der Bezugspunkte 36a und 38a relativ zu der Erfassung- und Anzeigeeinrichtung 52a ermittelt, wie vorstehend mit Bezug auf Fig.1 allgemein erläutert, die Verarbeitungseinrichtung 42 (in Fig.2 nicht gezeigt) das Kompromissdrehzentrum des Kniegelenks 12. Zusätzlich oder alternativ zu einer Ausgabe auf der Anzeigeeinheit 46 steuert die Verarbeitungseinrichtung 42 jedoch die Dioden 56a derart an, dass die beiden Lichtstrahlen 58a und 60a erzeugt werden, die sich in dem Punkt 48a treffen. Der Punkt 48a bildet die Projektion des aus den Messungen ermittelten Kompromissdrehzentrums auf das Kniegelenk 12. Der Orthopäde oder Orthopädietechniker, der die erfindungsgemäße Vorrichtung 10a einsetzt, erhält somit unmittelbar an dem Kniegelenk des Patienten das speziell für dieses Kniegelenk ermittelte Kompromissdrehzentrum durch den Kreuzungspunkt 48a angezeigt und kann dort eine farbliche Markierung auf der Haut des Patienten anbringen. Durch diese exakte patientenabhängige Bestimmung des Kompromissdrehzentrums ist es in der Folge möglich, eine Orthese genau an den Patienten anzupassen, so dass diese in nahezu optimaler Weise das orthetisch zu versorgende Kniegelenk 12 mechanisch und funktionell stabilisieren kann.

Es ist selbstverständlich, dass die vorstehend erläuterte Vorgehensweise zum Einsatz der erfindungsgemäßen Vorrichtung, ebenso wie die nachstehend noch zu beschreibenden Ausführungs- und Anwendungsbeispiele sich in gleicher Weise bei von dem Kniegelenk abweichenden Gelenken der Gliedmaßen eines Patienten einsetzen lässt.

Es ist darauf hinzuweisen, dass die Leuchtdioden 56a bei einer vereinfachten Ausführungsform der Erfindung auch nur dazu dienen können, Koordinaten an den Koordinatenachsen des durch die rechteckige Durchgangsöffnung 54a aufgespannten Koordinatenkreuzes anzuzeigen. Ein Orthopädietechniker kann dann beispielsweise durch Anlagen eines Lineals oder einer Linealanordnung an die durch die Leuchtdioden 56a angezeigten Koordinaten den Kreuzungspunkt 48a selbst ermitteln. Dabei ist nicht erforderlich, dass die Dioden 56a gerichtete sich in dem Punkt 48a kreuzende Lichtstrahlen ausgeben.
Fig.3 zeigt nun eine zweite Ausführungsform der erfindungsgemäßen Vorrichtung. Wiederum werden für gleichwirkende oder gleichartige Komponenten dieselben Bezugszeichen verwendet, wie bei der vorstehenden Beschreibung, allerdings mit dem Kleinbuchstaben "b" nachgestellt.

Der Aufbau des ersten Kopplungselements 16b und der mit diesem fest verbundenen Erfassungs- und Anzeigeeinrichtung 52b entspricht hinsichtlich der Anordnung und Wirkungsweise der Dioden 56b im Wesentlichen dem Aufbau gemäß Fig.2. Allerdings ist die Erfassungs- und Anzeigeeinrichtung als Erfassungs- und Anzeigering 52b mit entsprechend ringförmig angeordneten Leuchtdioden ausgebildet. Ferner unterscheidet sich das zweite Ausführungsbeispiel gemäß Fig.3 von dem ersten Ausführungsbeispiel gemäß Fig.2 in der Art der Erfassung der Relativposition der beiden Kopplungselemente 16b und 26b zueinander. Bei der zweiten Ausführungsform gemäß Fig.3 ist das zweite Kopplungselement 26b nämlich mit einer Querstrebe 64b fest verbunden. An den freien Enden der Querstrebe 64b sind Drehgelenke 66b, 68b angebracht. Über die Drehgelenke 66b und 68b ist die Querstrebe 64b mit Verbindungsarmen 70b, 72b drehgelenkig verbunden. Die Verbindungsarme 70b und 72b sind teleskopierbar ausgebildet. Hierzu weisen die beiden Verbindungsarme 70b, 72b Teleskopbereiche 74b, 76b auf, die es möglich machen, die Verbindungsarme 70b und 72b in ihrer Länge zu verändern. An ihren von der Querstrebe 64b angewandten Enden sind die beiden Verbindungsarme 70b, 72b über ein weiteres Drehgelenk 78b gelenkig mit dem Erfassungs- und Anzeigering 52b verbunden.

Sowohl das Drehgelenk 78b als auch die beiden Teleskopbereiche 74b und 76b ermöglichen eine Erfassung der aktuellen Stellungen. So ist das Drehgelenk 78b im Beispielsfall mit einem Dreh-Potentiometer versehen, welches die augenblickliche Winkelstellung der beiden Verbindungsarme 70b und 72b zueinander und zu dem Erfassungs- und Anzeigering 52b erfasst und diese in nicht gezeigter Weise an die Verarbeitungseinrichtung 42 (siehe Fig.1) weitergibt.

Die beiden Teleskopbereiche 74b und 76b sind mit Linearpotentiometern versehen und ermöglichen so eine exakte Bestimmung der augenblicklichen Länge der teleskopierbaren Verbindungsarme 70b und 72b. Auch die Linearpotentiometer sind in nicht gezeigter Weise mit der Verarbeitungseinrichtung 42 verbunden und geben die augenblicklichen Messwerte bezüglich der Länge der Verbindungsarme 70b und 72b an diese aus.

Aufgrund der gleichbleibenden Länge der Querstrebe 64b und der mittels der Erfassungseinrichtungen am Drehgelenk 78b (Drehpotentiometer) und an den Teleskopbereichen 74b, 76b (Linearpotentiometer) lässt sich zu jedem Zeitpunkt die Relativposition des zweiten Kopplungselements 26b relativ zu dem ersten Kopplungselement 16b ermitteln. In gleicher Weise wie vorstehend mit Bezug auf Fig.1 und 2 erläutert, lässt sich somit die Bewegung der Enden der Querstrebe 64b, das heißt die Bewegung der als Bezugspunkte anzusehenden Drehgelenke 66b und 68b erfassen. Aus den erhaltenen Daten lässt sich wiederum ein für das zu vermessende Gelenk charakteristischer Punkt, insbesondere das Kompromissdrehzentrum 48b, ermitteln und in bereits beschriebener Weise über die Leuchtdioden 56b nach dem Fadenkreuzprinzip auf dem zu vermessenden Kniegelenk anzeigen.

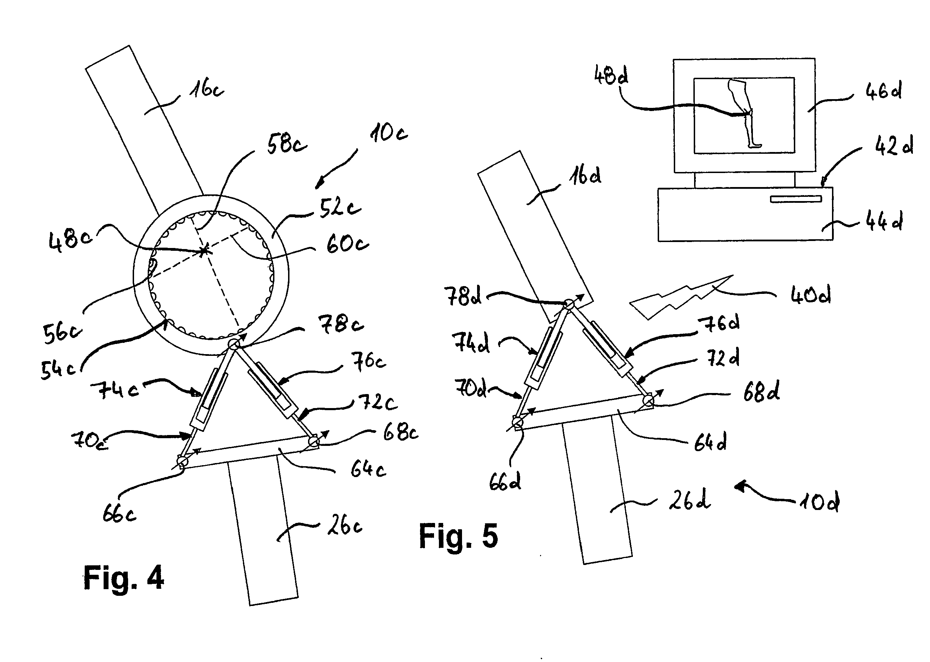

Fig.4 zeigt eine dritte Ausführungsform der erfindungsgemäßen Vorrichtung. Wiederum werden für gleichartige oder gleichwirkende Komponenten dieselben Bezugszeichen verwendet, jedoch mit dem Kleinbuchstaben "c" nachgestellt.

Die dritte Ausführungsform gemäß Fig.4 unterscheidet sich von der zweiten Ausführungsform gemäß Fig.3 lediglich darin, dass die beiden Teleskopbereiche 74c und 76c der Verbindungsarme 70c und 72c nicht mit Linearpotentiometern versehen sind. Stattdessen sind zusätzlich zu dem Drehgelenk 78c auch die beiden Drehgelenke 66c und 68c mit Drehpotentiometern ausgebildet, so dass sich über das Drehgelenk 66c der Winkel zwischen der Querstrebe 64c und dem Verbindungsarm 70c ermitteln lässt und mittels des Drehgelenks 68c der Winkel zwischen der Querstrebe 68c und dem Verbindungsarm 72c ermitteln lässt. Demnach lassen sich alle drei Winkel an den Drehgelenken 66c, 68c und 78c ermitteln. Darüber hinaus ist die Länge der Querstrebe 64c bekannt und bleibt unverändert. Somit lässt sich wiederum in einfacher Weise die Lage des Kopplungselements 26c relativ zu dem Kopplungselement 16c ermitteln und aus Lageveränderungen ein Kompromissdrehpunkt 48c berechnen, der in an sich bereits beschriebener Weise über die beiden sich kreuzenden Lichtstrahlen 58c und 60c auf dem Kniegelenk anzeigbar ist.

Fig.5 zeigt eine vierte Ausführungsform der erfindungsgemäßen Anordnung. Wiederum werden für gleichwirkende oder gleichartige Komponenten dieselben Bezugszeichen verwendet, wie bei der vorangehenden Beschreibung der Ausführungsbeispiele gemäß Fig.2 bis 4, jedoch mit dem Kleinbuchstaben "d" nachgestellt.

Bei der vierten Ausführungsform gemäß Fig.5 funktioniert die Erfassung der Relativlage der beiden Kopplungselemente 16d und 26d in im Wesentlich gleicher Weise, wie vorangehend mit Bezug auf Fig.4 erläutert. Über die in den Drehgelenken 66d, 68d und 78d angebrachten Drehpotentiometer lassen sich die Winkellagen der beiden Verbindungsarme 70d und 72d relativ zu dem Kopplungselement 16d, die Winkellagen zwischen den beiden Verbindungsarmen 70d und 72d sowie die Winkellagen der beiden Verbindungsarme 70d und 72d relativ zu der Querstrebe 64d ermitteln. Aufgrund der bekannten Länge der Querstrebe 64d lässt sich dann die Relativlage der beiden Drehgelenke 66d und 68d relativ zu dem Kopplungselement 16d ermitteln.

Allerdings erfolgt die Ausgabe nicht direkt am Kniegelenk, wie mit Bezug auf Fig.2 bis 4 erläutert. Vielmehr erfolgt eine Datenübertragung über eine in diesem Fall kabellos ausgebildete Schnittstelle 40d zu der Verarbeitungseinrichtung 42d. Dort wird dann der charakteristische Punkt, insbesondere das Kompromissdrehzentrum 48d, für das Kniegelenk ermittelt und an dem Bildschirm 46d ausgegeben. Diese Daten können dann der weiteren Dokumentation oder aber auch der manuellen oder automatischen Einstellung einer Orthese dienen. Gleichermaßen ist es möglich, dass auf die in Fig. 5 gezeigte Art und Weise Daten über ein gesundes Kniegelenk gewonnen werden und diese zur Einstellung einer Prothese für das prothetisch zu versorgende andere Bein des Patienten ausgewertet und genutzt werden.

Die Schnittstelle 40d kann beispielsweise als Infrarotschnittstelle, Bluetooth-Schnittstelle oder anderweitige kabellose Schnittstelle ausgebildet sein. Selbstverständlich ist es ebenso möglich, wie bereits in Fig.1 schematisch gezeigt, die Vorrichtung 10d über ein Verbindungskabel mit der Verarbeitungseinrichtung 42d zu koppeln.

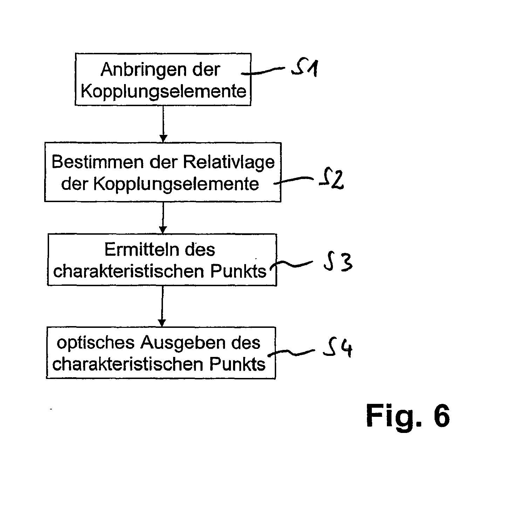

Fig.6 zeigt durch ein schematisches Ablaufdiagramm die Vorgehensweise beim Einsatz der erfindungsgemäßen Vorrichtung. Zunächst werden in Schritt S1 die beiden Kopplungselemente 16 und 26 an den Gliedern des orthopädisch zu vermessenden Gelenks angebracht. So dann wird in Schritt S2 über die Messeinrichtung die Relativlage der Kopplungselemente 16 und 26 zueinander ermittelt. Aus dieser Relativlage der Köpplungselemente 16 und 26 lässt sich dann in Schritt S3 ein charakteristischer Punkt, beispielsweise ein Kompromissdrehzentrum, für das jeweilige Gelenk ermitteln. Dieses wird dann in Schritt S4 über eine entsprechende optische Anordnung, beispielsweise ein LED-Array nach dem vorstehend geschilderten Fadenkreuzprinzip oder anderweitig, beispielsweise über einen Laser oder dergleichen, ausgegeben, so dass unmittelbar an dem orthopädisch zu vermessenden Gelenk der charakteristische Punkt angezeigt werden kann.

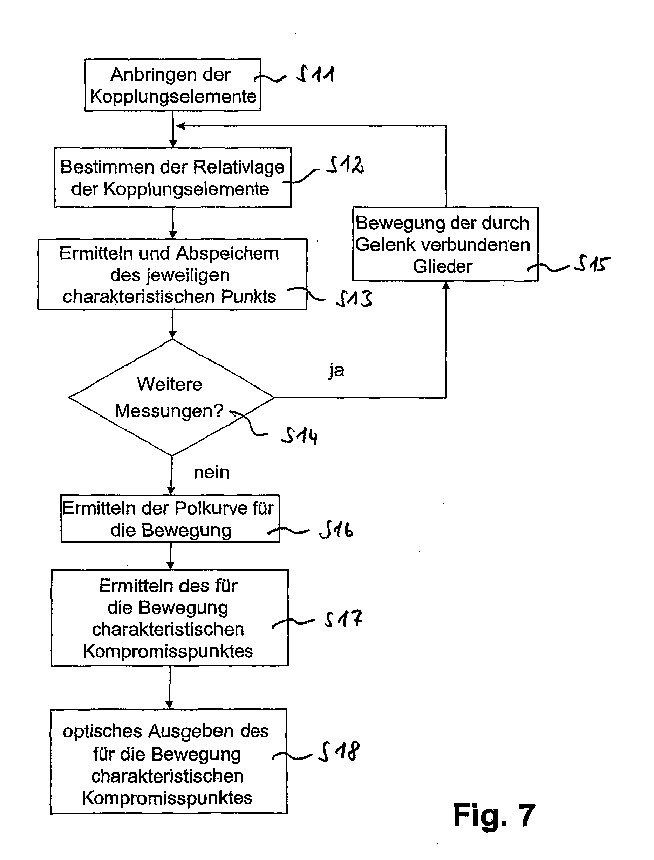

Fig.7 zeigt den Einsatz der erfindungsgemäßen Vorrichtung 10 in abgewandelter Form. Zunächst wird, wie bereits für Fig.6 erläutert, in Schritt S11 die Vorrichtung 10 an den Gliedern des zu vermessenden Gelenks angebracht, indem die beiden Kopplungselemente 16 und 26 an diesen befestigt werden. Sodann wird in Schritt S12 wiederum die Relativlage der Kopplungselemente 16 und 26 zueinander bestimmt. Daraus wird der jeweilige charakteristische Punkt, beispielsweise das Kompromissdrehzentrum, ermittelt und abgespeichert, wie in Schritt S13 gezeigt. In Schritt S14 erfolgt eine Abfrage, ob weitere Messungen durchgeführt werden sollen. Ist dies der Fall, so wird das Gelenk 12 bewegt, so dass sich die durch das Gelenk 12 verbundenen Glieder 18, 32 zueinander bewegen und damit auch die Kopplungselemente 16, 26 zueinander bewegen. Erneut wird die Relativlage der Kopplungselemente gemäß Schritt S12 zueinander bestimmt und, wie in Schritt S13 gezeigt, der jeweilige charakteristische Punkt ermittelt und abgespeichert, gegebenenfalls erfolgen weitere Messungen, wobei die Schleife aus den Schritten S12, S13, S14, S15 mehrfach durchlaufen wird.

Ist der Messvorgang abgeschlossen, so erfolgt kein erneuter Durchlauf durch die Schleife. Vielmehr wird dann aus den ermittelten und abgespeicherten charakteristischen Punkten für die ausgeführte Bewegung eine Polkurve ermittelt, wie in Schritt S16 gezeigt. Diese Polkurve zeigt den Verlauf der charakteristischen Punkte, im Spezialfall der Momentandrehpunkte, der beiden Glieder zueinander zu den verschiedenen Messzeitpunkten.

Aus dieser Polkurve kann dann gemäß Schritt S17 ein charakteristischer Kompromisspunkt für die erfasste Gelenkbewegung ermittelt werden, beispielsweise ein Kompromissdrehzentrum. Dieses Kompromissdrehzentrum wird beispielsweise derart gewählt, dass bei Verwendung einer Orthese mit einem monozentrischen Gelenk zur orthetischen Versorgung des vermessenen Gelenks die Drehachse des monozentrischen Orthesengelenks durch den ermittelten charakteristischen Kompromisspunkt verläuft und so die Orthese das vermessene Gelenk in bestmöglicher Weise stabilisiert, ohne dieses zusätzlich zu belasten.

Nach Ermittlung des charakteristischen Kompromisspunktes aus der Polkurve kann dieser wiederum gemäß Schritt S18 optisch ausgegeben werden, beispielsweise nach dem vorstehend mehrfach erläuterten Fadenkreuzprinzip über ein LED-Array oder an der Verarbeitungseinrichtung 42.

## Patentansprüche

1. Vorrichtung (10; 10a; 10b; 10c; 10d) zum Bestimmen wenigstens eines charakteristischen Punktes (48) eines orthopädisch zu vermessenden, zwei Glieder (18, 32) miteinander verbindenden Gelenks (12), wobei Vorrichtung (10; 10a; 10b; 10c; 10d) umfasst:
- ein erstes Kopplungselement (16), das an einem an das Gelenk (12) angrenzenden ersten Glied (18) anbringbar ist,
- ein relativ zu dem ersten Kopplungselement (16) bewegbares zweites Kopplungselement (26), das an einem an das Gelenk (12) angrenzenden zweiten Glied (32) anbringbar ist, und
- eine Messeinrichtung (24, 34) zum Bestimmen der Lage des ersten Kopplungselements (16) relativ zu dem zweiten Kopplungselement (26),
wobei die Messeinrichtung (24, 36) wenigstens einen Bezugspunkt (36, 38) aufweist, der fest mit einem (26) von erstem und zweitem Kopplungselement (16, 26) verbunden ist, und ferner eine Bezugspunkt-Erfassungs-einrichtung (24) aufweist, die fest mit dem jeweils anderen (16) von erstem und zweitem Kopplungselement (16, 26) verbunden ist,
**dadurch gekennzeichnet, dass** die Bezugspunkt-Erfassungseinrichtung (24) bei einer Bewegung von erstem und zweitem Glied (18, 32) zueinander eine Mehrzahl von Positionen des Bezugspunkts (36, 38) relativ zu der BezugspunktErfassungseinrichtung (24) erfasst und daraus den wenigstens einen charakteristischen Punkt (48) als einen virtuellen Drehpunkt für das zu vermessende Gelenk (12) ermittelt.

2. Vorrichtung (10; 10a; 10b; 10c; 10d) zum Bestimmen einer Mehrzahl charakteristischer Punkte eines orthopädisch zu vermessenden, zwei Glieder (18, 32) miteinander verbindenden Gelenks (12), wobei Vorrichtung (10; 10a; 10b; 10c; 10d) umfasst:
- ein erstes Kopplungselement (16), das an einem an das Gelenk (12) angrenzenden ersten Glied (18) anbringbar ist,
- ein relativ zu dem ersten Kopplungselement (16) bewegbares zweites Kopplungselement (26), das an einem an das Gelenk (12) angrenzenden zweiten Glied (32) anbringbar ist, und
- eine Messeinrichtung (24, 34) zum Bestimmen der Lage des ersten Kopplungselements (16) relativ zu dem zweiten Kopplungselement (26),
wobei die Messeinrichtung (24, 36) wenigstens einen Bezugspunkt (36, 38) aufweist, der fest mit einem (26) von erstem und zweitem Kopplungselement (16, 26) verbunden ist, und ferner eine Bezugspunkt-Erfassungseinrichtung (24) aufweist, die fest mit dem jeweils anderen (16) von erstem und zweitem Kopplungselement (16, 26) verbunden ist,
**dadurch gekennzeichnet, dass** die Bezugspunkt-Erfassungseinrichtung (24) bei einer Bewegung von erstem und zweitem Glied (18, 32) zueinander eine Mehrzahl von Positionen des Bezugspunkts (36, 38) relativ zu der Bezugspunkt-Erfassungseinrichtung (24) erfasst und daraus die Mehrzahl charakteristischer Punkte als eine Polkurve für das zu vermessende Gelenk (12) ermittelt.

3. Vorrichtung (10) nach Anspruch 1 oder 2,
**dadurch gekennzeichnet, dass** die Bezugspunkt-Erfassungseinrichtung (24) aus der Mehrzahl von Positionen des Bezugspunkts (36, 38) eine Bewegungsbahn des jeweiligen Bezugspunkts (36, 38) erfasst.

4. Vorrichtung (10) nach einem der Anspruch 1 bis 3,
**gekennzeichnet durch** eine Verarbeitungseinrichtung (42), die mit der Bezugspunkt-Erfassungseinrichtung (24) zur Übermittlung von Daten gekoppelt oder koppelbar ist.

5. Vorrichtung nach Anspruch 4,
**dadurch gekennzeichnet, dass** die Verarbeitungseinrichtung integral mit der Vorrichtung ausgebildet ist.

6. Vorrichtung (10) nach Anspruch 4 oder 5,
**dadurch gekennzeichnet, dass** die Verarbeitungseinrichtung (42) aus den von der Bezugspunkt-Erfassungseinrichtung (24) erhaltenen Daten, insbesondere aus der Bewegungsbahn des jeweiligen Bezugspunkts (36, 38), den wenigstens einen charakteristischen Punkt (48) oder die Mehrzahl charakteristischer Punkte ermittelt.

7. Vorrichtung (10) nach einem der vorangehenden Ansprüche,
**dadurch gekennzeichnet, dass** die Bezugspunkt-Erfassungseinrichtung (24) optisch die Position des wenigstens einen Bezugspunkts (48) erfasst.

8. Vorrichtung (10) nach einem der vorangehenden Ansprüche,
**dadurch gekennzeichnet, dass** die Bezugspunkt-Erfassungseinrichtung (24) elektronisch oder elektromagnetisch die Position des wenigstens einen Bezugspunkts (48) erfasst.

9. Vorrichtung (10) nach einem der vorangehenden Ansprüche,
**gekennzeichnet durch** wenigstens zwei voneinander beabstandete Bezugspunkte (36, 38), die fest mit dem einen (26) von erstem und zweitem Kopplungselement (16, 26) verbunden sind, wobei die Bezugspunkt-Erfassungseinrichtung (24) bei einer Bewegung von erstem und zweitem Glied (18, 32) zueinander eine Mehrzahl von Positionen der wenigstens zwei Bezugspunkte (36, 38), vorzugsweise die Bewegungsbahnen der wenigstens zwei Bezugspunkte (36, 38), relativ zu der Bezugspunkt-Erfassungseinrichtung (24) erfasst.

10. Vorrichtung (10) nach einem der vorangehenden Ansprüche,
**gekennzeichnet durch** eine optische Ausgabeeinrichtung (56a; 56b; 56c; 46d), mittels der der wenigstens eine ermittelte charakteristische Punkt (48) des Gelenks (12) optisch an dem zu vermessenden Gelenk (12) anzeigbar ist.

11. Vorrichtung (10) nach Anspruch 10,
**dadurch gekennzeichnet, dass** die Ausgabeeinrichtung eine gewinkelte Anordnung einer Mehrzahl von Lichtquellen (56a; 56b; 56c) umfasst, mittels denen vorzugsweise gerichtete Lichtstrahlen (58a, 60a; 58b, 60b; 58c, 60c) aussendbar sind, wobei der wenigstens eine ermittelte virtuelle Drehpunkt (48a; 48b; 48c) durch einem Kreuzungspunkt der beiden Lichtstrahlen (58a, 60a; 58b, 60b; 58c, 60c) anzeigbar ist.

12. Vorrichtung nach Anspruch 10 oder 11,
**dadurch gekennzeichnet, dass** die Ausgabeeinrichtung eine Anordnung einer Mehrzahl von Lichtquellen aufweist, die Anlagepunkte insbesondere zum Anlegen einer Lehrenanordnung, insbesondere einer Linealanordnung, anzeigen, wobei durch entsprechendes Anlegen der Lehrenanordnung oder per Augenmaß der wenigstens eine ermittelte virtuelle Drehpunkt an dem Gelenk antragbar ist.

13. Vorrichtung nach einem der vorangehenden Ansprüche,
**gekennzeichnet durch** eine elektromechanische Ausgabeeinrichtung, die eine elektromechanisch verlagerbare Markierung aufweist, wobei der wenigstens eine ermittelte virtuelle Drehpunkt **durch** entsprechende Positionierung der elektromechanisch verlagerbaren Markierung an dem Gelenk anzeigbar ist.

14. Vorrichtung nach Anspruch 13,
**gekennzeichnet durch** eine Markiervorrichtung, insbesondere einen Stift, mittels der an dem wenigstens einen ermittelten virtuellen Drehpunkt nach entsprechender elektromechanischer Verlagerung der Markierung an dem Gelenk eine Farbmarkierung anbringbar ist.

15. Verfahren zum Bestimmen wenigstens eines charakteristischen Punktes (48) eines orthopädisch zu vermessenden, zwei Glieder (18, 32) miteinander verbindenden Gelenks, unter Verwendung einer Vorrichtung (10; 10a; 10b; 10c; 10d), insbesondere nach einem der vorangehenden Ansprüche, wobei das Verfahren die Schritte umfasst:
- Anbringen eines ersten Kopplungselements (16) an einem an das Gelenk (12) angrenzenden ersten Glied (18),
- Anbringen eines relativ zu dem ersten Kopplungselement (16) bewegbaren zweiten Kopplungselements (26) an einem an das Gelenk (12) angrenzenden zweiten Glied (32) und
- Bestimmen der Lage des ersten Kopplungselements (16) relativ zu dem zweiten Kopplungselement (26) mittels einer Messeinrichtung (24, 34),
wobei die Messeinrichtung (24, 34) wenigstens einen Bezugspunkt (36, 38) aufweist, der fest mit einem (26) von erstem und zweitem Kopplungselement (16, 26) verbunden ist, und ferner eine BezugspunktErfassungs-einrichtung (24) aufweist, die fest mit dem jeweils anderen (26) von erstem und zweitem Kopplungselement (16, 26) verbunden ist,
**dadurch gekennzeichnet, dass** durch die Bezugspunkt-Erfassungseinrichtung (24) bei einer Bewegung von erstem und zweitem Glied (18, 32) zueinander eine Mehrzahl von Positionen des Bezugspunkts (36, 38) relativ zu der Bezugspunkt-Erfassungseinrichtung (24) erfasst werden und daraus der wenigstens eine charakteristische Punkt (48) als wenigstens ein virtueller Drehpunkt für das zu vermessende Gelenk (12) ermittelt wird.

16. Verfahren zum Bestimmen einer Mehrzahl charakteristischer Punkte eines orthopädisch zu vermessenden, zwei Glieder (18, 32) miteinander verbindenden Gelenks, unter Verwendung einer Vorrichtung (10; 10a; 10b; 10c; 10d), insbesondere nach einem der vorangehenden Ansprüche, wobei das Verfahren die Schritte umfasst:
- Anbringen eines ersten Kopplungselements (16) an einem an das Gelenk (12) angrenzenden ersten Glied (18),
- Anbringen eines relativ zu dem ersten Kopplungselement (16) bewegbaren zweiten Kopplungselements (26) an einem an das Gelenk (12) angrenzenden zweiten Glied (32) und
- Bestimmen der Lage des ersten Kopplungselements (16) relativ zu dem zweiten Kopplungselement (26) mittels einer Messeinrichtung (24, 34),
wobei die Messeinrichtung (24, 34) wenigstens einen Bezugspunkt (36, 38) aufweist, der fest mit einem (26) von erstem und zweitem Kopplungselement (16, 26) verbunden ist, und ferner eine Bezugspunkt-Erfassungseinrichtung (24) aufweist, die fest mit dem jeweils anderen (26) von erstem und zweitem Kopplungselement (16, 26) verbunden ist,
**dadurch gekennzeichnet, dass** durch die Bezugspunkt-Erfassungseinrichtung (24) bei einer Bewegung von erstem und zweitem Glied (18, 32) zueinander eine Mehrzahl von Positionen des Bezugspunkts (36, 38) relativ zu der Bezugspunkt-Erfassungseinrichtung (24) erfasst werden und in einem weiteren Schritt daraus die Mehrzahl charakteristischer Punkte als eine Polkurve für das zu vermessende Gelenk (12) ermittelt wird.

17. Verfahren nach Anspruch 15 oder 16,
**gekennzeichnet durch** den Schritt Erfassen einer Bewegungsbahn des jeweiligen Bezugspunktes (36, 38) aus der Mehrzahl von Positionen des Bezugspunkts (36, 38) durch die Bezugspunkt-Erfassungseinrichtung (24).

18. Verfahren nach einem der Ansprüche 15 bis 17,
**gekennzeichnet durch** den Schritt Ermitteln des wenigstens einen charakteristischen Punkts (48) oder der Mehrzahl charakteristischer Punkte aus den von der Bezugspunkt-Erfassungseinrichtung (24) erhaltenen Daten, insbesondere aus der Bewegungsbahn des jeweiligen Bezugspunkts (36, 38).

19. Verfahren nach einem der Ansprüche 15 bis 18,
**dadurch gekennzeichnet, dass** wenigstens zwei voneinander beabstandete Bezugspunkte (36, 38) verwendet werden, die fest mit dem einen (26) von erstem und zweitem Kopplungselement (16, 26) verbunden sind, wobei bei einer Bewegung von erstem und zweitem Glied (18, 32) zueinander eine Mehrzahl von Positionen der wenigstens zwei Bezugspunkte (36, 38), vorzugsweise die Bewegungsbahnen der wenigstens zwei Bezugspunkte (36, 38), relativ zu der Bezugspunkt-Erfassungseinrichtung (24) erfasst werden.

20. Verfahren nach einem der Ansprüche 15 bis 19,
**dadurch gekennzeichnet, dass** der wenigstens eine ermittelte charakteristische Punkt (48) des Gelenks (12) optisch oder durch eine elektromechanisch verlagerbare Markierung oder durch eine Farbmarkierung an dem zu vermessenden Gelenk (12) angezeigt wird.

## Claims

1. Device (10; 10a;. 10b; 10c; 10d) for determining at least one characteristic point (48) of a joint (12) that connects two limbs (18, 32) together and is to be measured orthopaedically, wherein the device (10; 10a; 10b; 10c; 10d) comprises:
- a first coupling element (16) which can be attached to a first limb (18) adjoining the joint (12),
- a second coupling element (26) which is movable relative to the first coupling element (16) and can be attached to a second limb (32) adjoining the joint (12), and
- a measuring device (24, 34) for determining the position of the first coupling element (16) relative to the second coupling element (26),
wherein the measuring device (24, 36) has at least one reference point (36, 38) which is fixedly connected to one (26) of the first and second coupling elements (16, 26), and further has a reference point detection device (24) which is fixedly connected to the other (16) of the first and second coupling elements (16, 26), **characterised in that**, when the first and second limbs (18, 32) move relative to one another, the reference point detection device (24) detects a plurality of positions of the reference point (36, 38) relative to the reference point detection device (24) and determines therefrom the at least one characteristic point (48) as a virtual pivot point for the joint (12) to be measured.

2. Device (10; 10a; 10b; 10c; 10d) for determining a plurality of characteristic points of a joint (12) that connects two limbs (18, 32) together and is to be measured orthopaedically, wherein the device (10; 10a; 10b; 10c; 10d) comprises:
- a first coupling element (16) which can be attached to a first limb (18) adjoining the joint (12),
- a second coupling element (26) which is movable relative to the first coupling element (16) and can be attached to a second limb (32) adjoining the joint (12), and
- a measuring device (24, 34) for determining the position of the first coupling element (16) relative to the second coupling element (26),
wherein the measuring device (24, 36) has at least one reference point (36, 38) which is fixedly connected to one (26) of the first and second coupling elements (16, 26), and further has a reference point detection device (24) which is fixedly connected to the other (16) of the first and second coupling elements (16, 26), **characterised in that**, when the first and second limbs (18, 32) move relative to one another, the reference point detection device (24) detects a plurality of positions of the reference point (36, 38) relative to the reference point detection device (24) and determines therefrom the plurality of characteristic points as a polar curve for the joint (12) to be measured.

3. Device (10) according to claim 1 or 2,
**characterised in that** the reference point detection device (24) detects a movement path of the reference point (36, 38) in question from the plurality of positions of the reference point (36, 38).

4. Device (10) according to any one of claims 1 to 3,
**characterised by** a processing system (42) which is coupled or can be coupled to the reference point detection device (24) for the transmission of data.

5. Device according to claim 4,
**characterised in that** the processing system is integral with the device.

6. Device (10) according to claim 4 or 5,
**characterised in that** the processing system (42) determines the at least one characteristic point (48) or the plurality of characteristic points from the data received from the reference point detection device (24), in particular from the movement path of the reference point (36, 38) in question.

7. Device (10) according to any one of the preceding claims,
**characterised in that** the reference point detection device (24) detects the position of the at least one reference point (48) optically.

8. Device (10) according to any one of the preceding claims,
**characterised in that** the reference point detection device (24) detects the position of the at least one reference point (48) electronically or electromagnetically.

9. Device (10) according to any one of the preceding claims,
**characterised by** at least two reference points (36, 38) which are spaced apart from one another and are fixedly connected to one (26) of the first and second coupling elements (16, 26), wherein, when the first and second limbs (18, 32) move relative to one another, the reference point detection device (24) detects a plurality of positions of the at least two reference points (36, 38), preferably the movement paths of the at least two reference points (36, 38), relative to the reference point detection device (24).

10. Device (10) according to any one of the preceding claims,
**characterised by** an optical output device (56a; 56b; 56c; 46d), by means of which the at least one determined characteristic point (48) of the joint (12) can be indicated optically on the joint (12) to be measured.

11. Device (10) according to claim 10,
**characterised in that** the output device comprises an angled arrangement of a plurality of light sources (56a; 56b; 56c), by means of which preferably directed light beams (58a, 60a; 58b, 60b; 58c, 60c) can be emitted, wherein the at least one determined virtual pivot point (48a; 48b; 48c) can be indicated by a point of intersection of the two light beams (58a, 60a; 58b, 60b; 58c, 60c).

12. Device according to claim 10 or 11,
**characterised in that** the output device has an arrangement of a plurality of light sources which indicate contact points, in particular for the positioning of a gauge arrangement, in particular a ruler arrangement, wherein the at least one determined virtual pivot point can be marked out on the joint by appropriate positioning of the gauge arrangement or by visual judgment.

13. Device according to any one of the preceding claims,
**characterised by** an electromechanical output device which has an electromechanically displaceable marking, wherein the at least one determined virtual pivot point can be indicated on the joint by appropriate positioning of the electromechanically displaceable marking.

14. Device according to claim 13,
**characterised by** a marking device, in particular a pen, by means of which a coloured marking can be applied to the joint at the at least one determined virtual pivot point after appropriate electromechanical displacement of the marking.

15. Method for determining at least one characteristic point (48) of a joint that connects two limbs (18, 32) together and is to be measured orthopaedically, using a device (10; 10a; 10b; 10c; 10d), in particular according to any one of the preceding claims, wherein the method comprises the steps:
- applying a first coupling element (16) to a first limb (18) adjoining the joint (12),
- applying a second coupling element (26), which is movable relative to the first coupling element (16), to a second limb (32) adjoining the joint (12), and
- determining the position of the first coupling element (16) relative to the second coupling element (26) by means of a measuring device (24, 34),
wherein the measuring device (24, 34) has at least one reference point (36, 38) which is fixedly connected to one (26) of the first and second coupling elements (16, 26), and further has a reference point detection device (24) which is fixedly connected to the other (26) of the first and second coupling elements (16, 26), **characterised in that**, when the first and second limbs (18, 32) move relative to one another, a plurality of positions of the reference point (36, 38) relative to the reference point detection device (24) are detected by the reference point detection device (24), and the at least one characteristic point (48) is determined therefrom as at least one virtual pivot point for the joint (12) to be measured.

16. Method for determining a plurality of characteristic points of a joint that connects two limbs (18, 32) together and is to be measured orthopaedically, using a device (10; 10a; 10b; 10c; 10d), in particular according to any one of the preceding claims, wherein the method comprises the steps:
- applying a first coupling element (16) to a first limb (18) adjoining the joint (12),
- applying a second coupling element (26), which is movable relative to the first coupling element (16), to a second limb (32) adjoining the joint (12), and
- determining the position of the first coupling element (16) relative to the second coupling element (26) by means of a measuring device (24, 34),
wherein the measuring device (24, 34) has at least one reference point (36, 38) which is fixedly connected to one (26) of the first and second coupling elements (16, 26), and further has a reference point detection device (24) which is fixedly connected to the other (26) of the first and second coupling elements (16, 26), **characterised in that**, when the first and second limbs (18, 32) move relative to one another, a plurality of positions of the reference point (36, 38) relative to the reference point detection device (24) are detected by the reference point detection device (24), and in a further step the plurality of characteristic points are determined therefrom as a polar curve for the joint (12) to be measured.

17. Method according to claim 15 or 16,
**characterised by** the step of detecting a movement path of the reference point (36, 38) in question from the plurality of positions of the reference point (36, 38) by the reference point detection device (24).

18. Method according to any one of claims 15 to 17,
**characterised by** the step of determining the at least one characteristic point (48) or the plurality of characteristic points from the data received from the reference point detection device (24), in particular from the movement path of the reference point (36, 38) in question.

19. Method according to any one of claims 15 to 18,
**characterised in that** there are used at least two reference points (36, 38) which are spaced apart from one another and are fixedly connected to one (26) of the first and second coupling elements (16, 26), wherein, when the first and second limbs (18, 32) move relative to one another, a plurality of positions of the at least two reference points (36, 38), preferably the movement paths of the at least two reference points (36, 38), relative to the reference point detection device (24) are detected.

20. Method according to any one of claims 15 to 19,
**characterised in that** the at least one determined characteristic point (48) of the joint (12) is indicated on the joint (12) to be measured optically or by an electromechanically displaceable marking or by a coloured marking.

## Revendications

1. Dispositif (10 ; 10a ; 10b ; 10c ; 10d) permettant de déterminer au moins un point caractéristique (48) d'une articulation (12) à mesurer par voie orthopédique et reliant l'un à l'autre deux membres (18, 32), ledit dispositif (10 ; 10a ; 10b ; 10c ; 10d) comprenant :
- un premier élément de couplage (16) pouvant être placé sur un premier membre (18) adjacent à l'articulation (12),
- un deuxième élément de couplage (26) mobile par rapport audit premier élément de couplage (16) et pouvant être placé sur un deuxième membre (32) adjacent à l'articulation (12), et
- un dispositif de mesure (24, 34) permettant de déterminer la position du premier élément de couplage (16) par rapport audit deuxième élément de couplage (26),
ce dispositif de mesure (24, 34) présentant au moins un point de référence (36, 38) qui est solidaire de l'un (26) desdits premier et deuxième éléments de couplage (16, 26), ainsi qu'un dispositif de saisie de point de repère (24) qui est solidaire de l'autre (16) desdits premier et deuxième éléments de couplage (16, 26),
**caractérisé en ce que** lors d'un déplacement du premier et du deuxième membre (18, 32) l'un par rapport à l'autre, ce dispositif de saisie de point de repère (24) saisit une pluralité de positions du point de référence (36, 38) par rapport au dispositif de saisie de point de référence (24) et détermine à partir de là au moins un point caractéristique (48) comme pivot virtuel pour l'articulation (12) à mesurer.

2. Dispositif (10 ; 10a ; 10b ; 10c ; 10d) permettant de déterminer une pluralité de points caractéristiques d'une articulation (12) à mesurer par voie orthopédique et reliant l'un à l'autre deux membres (18, 32), ce dispositif (10 ; 10a ; 10b ; 10c ; 10d) comprenant :
- un premier élément de couplage (16) pouvant être placé sur un premier membre (18) adjacent à l'articulation (12),
- un deuxième élément de couplage (26) mobile par rapport audit premier élément de couplage (16) et pouvant être placé sur un deuxième membre (32) adjacent à l'articulation (12), et
- un dispositif de mesure (24, 34) permettant de déterminer la position du premier élément de couplage (16) par rapport au deuxième élément de couplage (26),
ce dispositif de mesure (24, 36) présentant au moins un point de référence (36, 38) qui est solidaire de l'un (26) desdits premier et deuxième éléments de couplage (16, 26), ainsi qu'un dispositif de saisie de point de repère (24) qui est solidaire de l'autre (16) desdits premier et deuxième éléments de couplage (16, 26),
**caractérisé en ce que** lors d'un déplacement du premier et du deuxième membre (18, 32) l'un par rapport à l'autre, ce dispositif de saisie de point de repère (24) saisit une pluralité de positions du point de référence (36, 38) par rapport au dispositif de saisie de point de référence (24) et détermine à partir de là la pluralité de points caractéristiques (48) en tant que courbe polaire pour l'articulation (12) à mesurer.

3. Dispositif (10) selon la revendication 1 ou 2,
**caractérisé en ce que** le dispositif de saisie de point de repère (24) saisit à partir de la pluralité de positions du point de référence (36, 38) une trajectoire du point de référence (36, 38) respectif.

4. Dispositif (10) selon l'une des revendications 1 à 3,
**caractérisé par** un dispositif de traitement (42) couplé ou pouvant être couplé au dispositif de saisie de point de repère (24) pour la transmission de données.

5. Dispositif (10) selon la revendication 4,
**caractérisé en ce que** ledit dispositif de traitement (42) est solidairement réalisé avec le dispositif.

6. Dispositif (10) selon la revendication 4 ou 5,
**caractérisé en ce que** le dispositif de traitement (42) détermine à partir des données fournies par le dispositif de saisie de point de repère (24), en particulier à partir de la trajectoire du point de référence respectif (36, 38), au moins un point caractéristique (48) ou la pluralité de points caractéristiques.

7. Dispositif (10) selon l'une des revendications précédentes,
**caractérisé en ce que** le dispositif de saisie de point de repère (24) saisit optiquement la position dudit point de référence (48).

8. Dispositif (10) selon l'une des revendications précédentes,
**caractérisé en ce que** le dispositif de saisie de point de repère (24) saisit électroniquement ou électromagnétiquement la position dudit point de référence (48).

9. Dispositif (10) selon l'une des revendications précédentes,
**caractérisé par** au moins deux points de références (36, 38) qui sont espacés l'un de l'autre et solidaires de l'un (26) desdits premier et deuxième éléments de couplage (16, 26), ce dispositif de saisie de point de repère (24) saisissant une pluralité de positions desdits deux points de référence (36, 38), préférentiellement les trajectoires desdits deux points de référence (36, 38) par rapport au dispositif de saisie de point de référence (24), lors du déplacement du premier et du deuxième membre (18, 32) l'un par rapport à l'autre.

10. Dispositif (10) selon l'une des revendications précédentes,
**caractérisé par** un dispositif de sortie optique (56a ; 56b ; 56c ; 46d) au moyen duquel ledit point caractéristique (48) déterminé de l'articulation (12) peut être optiquement affiché sur l'articulation (12) à mesurer.

11. Dispositif (10) selon la revendication 10,
**caractérisé en ce que** ledit dispositif de sortie comprend un agencement angulaire formé par une pluralité de sources lumineuses (56a ; 56b ; 56c) au moyen desquelles des rayons lumineux préférentiellement collimatés (58a, 60a ; 58b, 60b ; 58, 60c) peuvent être émis, ledit pivot virtuel déterminé (48a ; 48b ; 48c) pouvant être affiché à l'aide d'un point d'intersection des deux rayons lumineux (58a, 60a ; 58b, 60b ; 58c, 60c).

12. Dispositif (10) selon la revendication 10 ou 11,
**caractérisé en ce que** le dispositif de sortie présente un agencement formé par une pluralité de sources lumineuses qui affichent les points d'appui, en particulier pour appliquer un agencement de gabarits, en particulier un agencement de règles, ledit pivot virtuel déterminé pouvant être porté sur l'articulation par application correspondante dudit agencement de gabarits ou à vue d'oeil.

13. Dispositif (10) selon l'une des revendications précédentes,
**caractérisé par** un dispositif de sortie électromécanique qui présente un marquage pouvant être déplacé électromécaniquement, ledit pivot virtuel déterminé pouvant être affiché sur l'articulation par positionnement correspondant du marquage déplaçable électromécaniquement.

14. Dispositif (10) selon la revendication 13,
**caractérisé par** un dispositif de marquage, en particulier un crayon, au moyen duquel un marquage de couleur peut être appliqué sur ledit pivot virtuel déterminé après déplacement électromécanique correspondant du marquage sur l'articulation.

15. Procédé permettant de déterminer au moins un point caractéristique (48) d'une articulation (12) à mesurer par voie orthopédique et reliant l'un à l'autre deux membres (18, 32), dans le cadre duquel il est utilisé un dispositif (10 ; 10a ; 10b ; 10c ; 10d), en particulier selon l'une des revendications précédentes, le procédé comportant les étapes suivantes :
- la mise en place d'un premier élément de couplage (16) sur un premier membre (18) adjacent à l'articulation (12),
- la mise en place d'un deuxième élément de couplage (26) mobile par rapport au premier élément de couplage (16) sur un deuxième membre (32) adjacent à l'articulation (12), et
- la détermination de la position du premier élément de couplage (16) par rapport au deuxième élément de couplage (26) au moyen d'un dispositif de mesure (24, 34),
ce dispositif de mesure (24, 34) présentant au moins un point de référence (36, 38) qui est solidaire de l'un (26) desdits premier et deuxième éléments de couplage (16, 26), ainsi qu'un dispositif de saisie de point de repère (24) qui est solidaire de l'autre (26) desdits premier et deuxième éléments de couplage (16, 26),
**caractérisé en ce que** lors d'un déplacement du premier et du deuxième membre (18, 32) l'un par rapport à l'autre, le dispositif de saisie de point de repère (24) saisit une pluralité de positions du point de référence (36, 38) par rapport au dispositif de saisie de point de référence (24) et détermine à partir de là au moins un point caractéristique (48) comme pivot virtuel pour l'articulation (12) à mesurer.

16. Procédé permettant de déterminer une pluralité de points caractéristiques d'une articulation à mesurer par voie orthopédique et reliant l'un à l'autre deux membres (18, 32), dans le cadre duquel il est utilisé un dispositif (10 ; 10a ; 10b ; 10c ; 10d), en particulier selon l'une des revendications précédentes, le procédé comportant les étapes suivantes :
- la mise en place d'un premier élément de couplage (16) sur un premier membre (18) adjacent à l'articulation (12),
- la mise en place d'un deuxième élément de couplage (26) mobile par rapport au premier élément de couplage (16) sur un deuxième membre (32) adjacent à l'articulation (12), et
- la détermination de la position du premier élément de couplage (16) par rapport au deuxième élément de couplage (26) au moyen d'un dispositif de mesure (24, 34),
ce dispositif de mesure (24, 34) présentant au moins un point de référence (36, 38) qui est solidaire de l'un (26) desdits premier et deuxième éléments de couplage (16, 26), ainsi qu'un dispositif de saisie de point de repère (24) qui est solidaire de l'autre (26) desdits premier et deuxième éléments de couplage (16, 26),
**caractérisé en ce que** lors d'un déplacement du premier et du deuxième membre (18, 32) l'un par rapport à l'autre, le dispositif de saisie de point de repère (24) saisit une pluralité de positions du point de référence (36, 38) par rapport au dispositif de saisie de point de référence (24) et détermine ensuite, à partir de là, la pluralité de points caractéristiques en tant que courbe polaire pour l'articulation (12) à mesurer.

17. Procédé (10) selon la revendication 15 ou 16,
**caractérisé par** l'étape consistant en la saisie, à partir de la pluralité de positions du point de référence (36, 38), d'une trajectoire du point de référence (36, 38) respectif par le dispositif de saisie de point de référence (24).

18. Procédé (10) selon l'une des revendications 15 à 17,
**caractérisé par** l'étape consistant en la détermination dudit point caractéristique (48) ou de la pluralité de points caractéristiques à partir des données fournies par le dispositif de saisie de point de repère (24), en particulier à partir de la trajectoire du point de référence (36, 38) respectif.

19. Procédé (10) selon l'une des revendications 15 à 18,
**caractérisé en ce que** sont utilisés au moins deux points de référence (36, 38) qui sont espacés l'un de l'autre et reliés solidairement à l'un (26) desdits premier et deuxième éléments de couplage (16, 26), dans le cadre duquel une pluralité de positions desdits deux points de référence (36, 38), préférentiellement les trajectoires de ceux-ci, sont saisis par rapport au dispositif de saisie de point de repère (24).

20. Procédé (10) selon l'une des revendications 15 à 19,
**caractérisé en ce que** ledit point caractéristique déterminé (48) de l'articulation (12) est affiché optiquement ou par marquage déplaçable électromécaniquement ou par marquage de couleur sur l'articulation (12) à mesurer.
